# EUROPEAN PATENT SPECIFICATION

(11) **EP 0 983 294 B1**
(45) Date of publication and mention of the grant of the patent: **08.01.2003**
(21) Application number: 98919932.8
(22) Date of filing: 28.04.1998
(51) Int. Cl.: C07J 31/00, A61K 31/565

(54) **B-RING ESTRATRIENE DIOL SULPHATES**
B-RING ESTRATRIENDIOL-SULFATE
ESTRATRIENE DIOL SULFATES A CYCLE B

(30) Priority: 05.05.1997 US 841882
(43) Date of publication of application: 08.03.2000
(73) Proprietor: Wyeth, Madison, New Jersey 07940-0874 (US)
(72) Inventor: KAGAN, Michael, Z., Plainsboro, NJ 08536 (US); RAVEENDRANATH, Panolil, Monroe, NY 10950 (US); SHAH, Syed, Muzafar, East Hanover, NJ 07936 (US); WINKLEY, Michael, William, Campbell Hall, NY 10916 (US)
(74) Representative: Talbott, Dawn Jacqueline
(86) International application number: US9808483
(87) International publication number: WO98050414

(56) References cited:
- DE-C- 635 781
- GB-A- 864 231
- R. JOHNSON ET AL: "Steady State Urinary Excretion Method for Determining Bioequivalence of Conjugated Estrogen Products" JOURNAL OF PHARMACEUTICAL SCIENCES., vol. 67, no. 9, September 1978, WASHINGTON US, pages 1218-1224, XP002070687
- L. STARKA ET AL: "Biosynthesis of Equilin and Equilenin from 7.alpha.-hydroxydehydroepiandrosterone and its Sulfate by Human Placenta Maintained in Organ Culture" J. EUR. STEROIDES, vol. 1, no. 1, 1966, pages 37-45, XP002072337
- W. E. BACHMANN ET AL: "195. Über die Spaltung der Doppelbindung im Neoergosterol" HELVETICA CHIMICA ACTA., vol. 42, no. 6, 15 October 1959, BASEL CH, pages 1790-1793, XP002070690
- A. H. EIMASRY ET AL: "19-Norsteroids of Unnatural Configuration from Erogesterol" JOURNAL OF PHARMACEUTICAL SCIENCES., vol. 59, no. 4, April 1970, WASHINGTON US, pages 449-458, XP002070691
- P. KOCOVSKY ET AL: "The Synthesis of Androstane Derivatives with an Aromatic B-RIng" COLLECTION OF CZECHOSLOVAK CHEMICAL COMMUNICATIONS., vol. 39, no. 7, 1974, PRAGUE CS, pages 1905-1913, XP002070689
- W. H. SCHULLER ET AL: "Conversion of Ergosterol into the Estrogen Neoergosterol by Direct Peroxide Cleavage" JOURNAL OF MEDICINAL CHEMISTRY., vol. 14, no. 5, May 1971, WASHINGTON US, page 466 XP002070692
- R. H. PETERS ET AL: "17-Desoxy Estrogen Analogues" JOURNAL OF MEDICINAL CHEMISTRY., vol. 32, no. 7, July 1989, WASHINGTON US, pages 1642-1652, XP002070693

## Description

The use of naturally occurring estrogenic compositions of substantial purity and low toxicity such as PREMARIN™ (conjugated equine estrogens) has become a preferred medical treatment for alleviating the symptoms of menopausal syndrome, osteoporosis/osteopenia in estrogen deficient women and in other hormone related disorders. The estrogenic components of the naturally occurring estrogenic compositions have been generally identified as sulfate esters of estrone, equilin, equilenin, 17-β-estradiol, dihydroequilenin and 17-β-dihydroequilenin (U.S. Patent 2,834,712). The estrogenic compositions are usually buffered or stabilized with alkali metal salts of organic or inorganic acids at a substantially neutral pH of about 6.5 to 7.5. Urea has also been used as a stabilizer (U.S. 3,608,077). The incorporation of antioxidants to stabilize synthetic conjugated estrogens and the failure of pH control with tris(hydroxymethyl)aminomethane (TRIS) to prevent hydrolysis is discussed in U.S. 4,154,820.

One of the compounds described herein, 5,7,9-estratriene-3β,17β-diol 3-sulfate ester sodium salt is a minor component of PREMARIN™ (conjugated equine estrogens).

### DESCRIPTION OF THE INVENTION

In accordance with this invention, there are provided estrogenic agents having the formula I 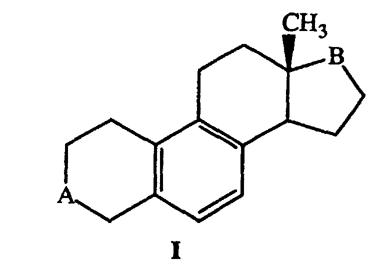 wherein
- A and B: are each, independently, 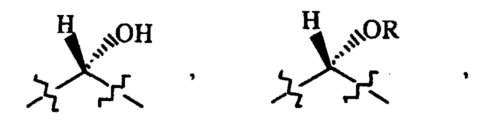 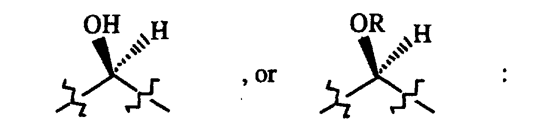
- R: is SO₃⁻X⁺;
- X⁺: is alkali metal, alkaline earth metal, ammonium, alkylammonium containing 1-6 carbon atoms, dialkylammonium containing 1-6 carbon atoms in each alkyl group, trialkylammonium containing 1-6 carbon atoms in each alkyl group or tetraalkylammonium containing 1-6 carbon atoms in each alkyl group;
with the proviso that at least one of A or B is

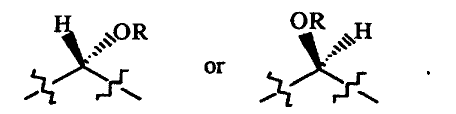

Alkali metal salts include sodium and potassium salts, particularly preferred are sodium salts. Alkaline earth metal salts include calcium and magnesium salts. Suitable alkyl groups include methyl, ethyl, propyl, butyl, pentyl and hexyl, preferred alkyl groups being methyl and ethyl. Where more than one alkyl group is present the groups may be the same or different. Preferred trialkylammonium salts are trimethylammonium salts and triethylammonium salts.

One embodiment of the invention is 5,7,9-Estratriene-3β,17β-diol 3-sulfate ester sodium salt.

This invention further provides a pharmaceutical composition which comprises a compound having the formula 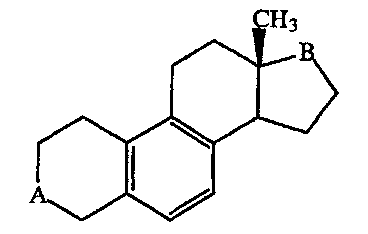 wherein
- A and B: are each, independently, 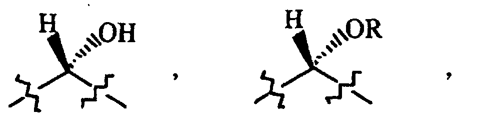 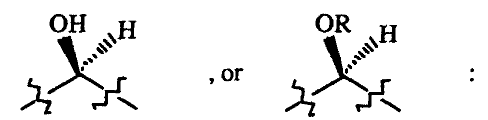
- R: is SO₃⁻X⁺;
- X⁺: is alkali metal, alkaline earth metal, ammonium, alkylammonium containing 1-6 carbon atoms, dialkylammonium containing 1-6 carbon atoms in each alkyl group, trialkylammonium containing 1-6 carbon atoms in each alkyl group or tetraalkylammonium containing 1-6 carbon atoms in each alkyl group;
with the proviso that at least one of A or B is

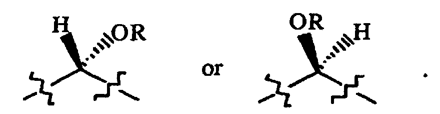

This invention also provides the above mentioned compounds for use as a medicament.

This invention additionally provides the use of a compound having the formula 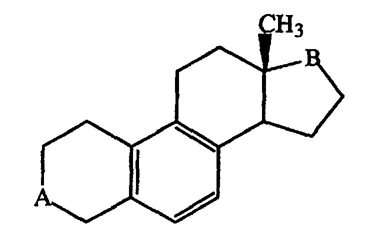 wherein
- A and B: are each, independently, 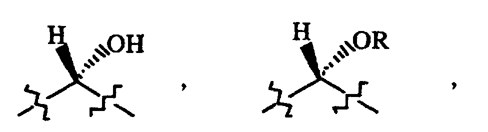 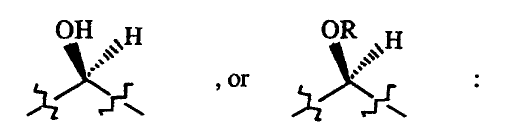
- R: is SO₃⁻X⁺;
- X⁺: is alkali metal, alkaline earth metal, ammonium, alkylammonium containing 1-6 carbon atoms, dialkylammonium containing 1-6 carbon atoms in each alkyl group, trialkylammonium containing 1-6 carbon atoms in each alkyl group or tetraalkylammonium containing 1-6 carbon atoms in each alkyl group;
with the proviso that at least one of A or B is 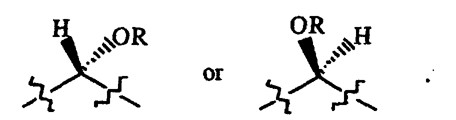 in the preparation of a medicament for providing estrogen replacement therapy or treating estrogen deficiency in a mammal.

As used in accordance with this invention, treating covers treatment of an existing condition, ameliorating the condition, or providing palliation of the condition and inhibiting includes inhibiting or preventing the progress or development of the condition.

An embodiment of this invention is the use of such a compound for treating vasomotor symptoms relating to estrogen deficiency in a mammal e.g. where the vasomotor symptom is hot flushes.

A further embodiment of this invention is the use of such a compound in the preparation of a medicament for treating or inhibiting osteoporosis or atherosclerosis in a mammal.

The present invention further provides compositions comprising a compound of formula I. In particular it provides compositions comprising at least 1% of a compound of formula I. One aspect of the present invention provides compositions wherein the only estrogenic agent is a compound of formula I. Embodiments of the present invention include compositions wherein the only active compound is a compound of formula I. In these embodiments other excipients and carriers may be included but no further active materials are included.

The present invention also provides processes for the preparation of the compounds described above. It provides processes for the preparation of 5,7,9-estratriene-3,17-diol-3,17-disulfate ester salts (i.e. compounds wherein A and B are each independently a sulphate ester salt) which comprise:
a) converting the appropriate 5,7,9-estratriene-3,17-diol-3,17-disulfate ester to a pharmaceutically acceptable salt,
b) converting a pharmaceutically acceptable salt of the 5,7,9-estratriene-3,17-diol-3,17-disulfate ester to a different pharmaceutically acceptable salt of the appropriate 5,7,9-estratriene-3,17-diol-3,17-disulfate ester or
c) directly converting the appropriate 5,7,9-estratriene-3,17-diol to the desired pharmaceutically acceptable salt of 5,7,9-estratriene-3,17-Bisulfate ester.

5,7,9-estratriene-3,17-diol-3,17-disulfate esters may be converted to pharmaceutically acceptable salts by neutralising the acid with an appropriate base, e.g. with an alkali metal carbonate, an alkaline earth metal carbonate or a primary, secondary, tertiary or quaternary amine carbonate. Alkali metal or alkaline earth metal salts may be prepared by using the appropriate alkali metal hydride e.g. sodium hydride, potassium hydride or lithium hydride.

A pharmaceutically acceptable salt of the appropriate 5,7,9-estratriene-3,17-diol-3,17-disulfate ester may be converted to a different pharmaceutically acceptable salt by displacement, by using an ion exchange resin or by double decomposition (metastasis). Displacement of a weak base with a stronger one may be utilised to convert, e.g. an amine salt to an alkali metal salt or an alkaline earth metal salt using an appropriate base, e.g. a hydroxide. For example a trialkylamine salt such as a triethylamine salt may be converted to an alkali metal salt such as a sodium salt by treating it with an alkali metal hydroxide such as aqueous sodium hydroxide. The displacement may be carried out using an ion exchange resin. Alternatively one salt may be converted to another by double decomposition, e.g. an alkaline earth metal salt such as the calcium salt may be replaced with an alkali metal salt. E.g. the calcium salt of the 5,7,9-estratriene-3,17-diol-3,17-disulfate ester may be dissolved in water followed by the addition of e.g. sodium carbonate. Insoluble calcium carbonate would then precipitate out to provide the sodium salt of the desired 5,7,9-estratriene-3,17-diol-3,17-disulfate ester.

A pharmaceutically acceptable salt of the desired 5,7,9-estratriene-3,17-diol-3,17-disulfate ester may be prepared by directly converting the appropriate 5,7,9-estratriene-3,17-diol. This may be performed by reacting it with the appropriate aminesulfurtrioxide complex, e.g. by reacting it with a trialkylaminesulfurtrioxide (such as triethylaminesulfurtrioxide complex) to provide the corresponding trialkylamine salt (such as the triethylamine salt). If desired the salt may then be converted to another salt of the invention as described above.

The invention also provides processes for the preparation of 5,7,9-estratriene-3,17-diol-3-sulfate ester salts or 5,7,9-estratriene-3,17-diol-17-sulfate ester salts (i.e. compounds wherein one of A and B is a sulphate ester salt and the other is hydrogen) which comprise:
a) converting the appropriate 5,7,9-estratriene-3,17-diol-mono sulfate ester to a pharmaceutically acceptable salt,
b) converting a pharmaceutically acceptable salt of the appropriate 5,7,9-estratriene-3,17-diol-mono sulfate ester to a different pharmaceutically acceptable salt of the 5,7,9-estratriene-3,17-diol-mono sulfate ester,
c) converting the corresponding 5,7,9-estratriene-3-ol,17-keto 3 sulfate ester salt or 5,7,9-estratriene-17-ol,3-keto 17 sulfate ester salt to the desired 5,7,9-estratriene-3,17-diol-3-sulfate ester salt or 5,7,9-estratriene-3,17-diol-17-sulfate ester salt
d) converting 5,7,9-estratriene-3,17-diol to the desired pharmaceutically acceptable salt of the desired 5,7,9-estratriene-3,17-diol-monosulfate ester,
e) deprotecting the corresponding 17 protected 5,7,9-estratriene-3,17-diol-3-sulfate ester salt or 3 protected 5,7,9-estratriene-3,17-diol-17-sulfate ester salt or
f) converting one stereo isomer of the pharmaceutically acceptable salt of the appropriate 5,7,9-estratriene-3,17-diol-mono sulfate ester to another by inversion.

The conversion of the appropriate 5,7,9-estratriene-3,17-diol-mono sulfate ester to a pharmaceutically acceptable salt and the conversion of a pharmaceutically acceptable salt of the appropriate 5,7,9-estratriene-3,17-diol-mono sulfate ester to a different pharmaceutically acceptable salt of the 5,7,9-estratriene-3,17-diol-mono sulfate ester may be performed by analogy to the methods described above.

5,7,9-estratriene-3-ol,17-keto mono sulfate ester salt may be converted to the desired 5,7,9-estratriene-3,17-diol-mono-sulfate ester salt by reduction with a suitable reducing agent, e.g. LiBH₄. The steric influence of the nearby methyl will cause some reducing agents to preferentially provide the 17β compounds. These can be converted to the corresponding α compounds by inversion, e.g. by a MITSUNOBU inversion.

5,7,9-estratriene-3,17-diol may be converted directly to the desired pharmaceutically acceptable salt of the desired 5,7,9-estratriene-3,17-diol-monosulfate ester by analogy to the methods described above.

In order to prepare the desired 5,7,9-estratriene-3-ol,17-keto mono sulfate ester salt it may be necessary to protect the second hydroxy group. This may be achieved by conventional means using a suitable protecting group which will finally be removed to provide the product.

In order to protect the less reactive 17 hydroxy group it may be necessary to first protect the 3 hydroxy group. For example the 3 hydroxy group may be protected with a first protecting group and then the 17 hydroxy with a second protecting group. The 3 hydroxy group may then be converted to the desired sulphate salt. The first protecting group may be removed before this conversion or at the same time by utilising a reagent which will replace the first protecting group with the sulphate group. The second protecting group may then be removed to provide the desired product. Alternatively the second protecting group may be removed at the same time the 3 hydroxy is converted to the sulphate salt.

The present invention also provides compounds of formula I prepared by a chemical process, particularly those prepared according to the processes described above. The invention also provides compounds of formula I obtainable by such processes.

The compounds of this invention can be prepared from readily available starting materials. The following schemes show the preparation of representative compounds of this invention. For example, 5,7,9-estratriene-3β,17β-diol 3-sulfate ester sodium salt (**7**) can be prepared from 3β-hydroxy-5,7,9-estratriene-17-one according to Scheme I. 3β-Hydroxy-5,7,9-estratriene-17-one can be prepared according to D. Banerjee in Ind. Chim. Beige. Suppl. 2: 435 (1959).

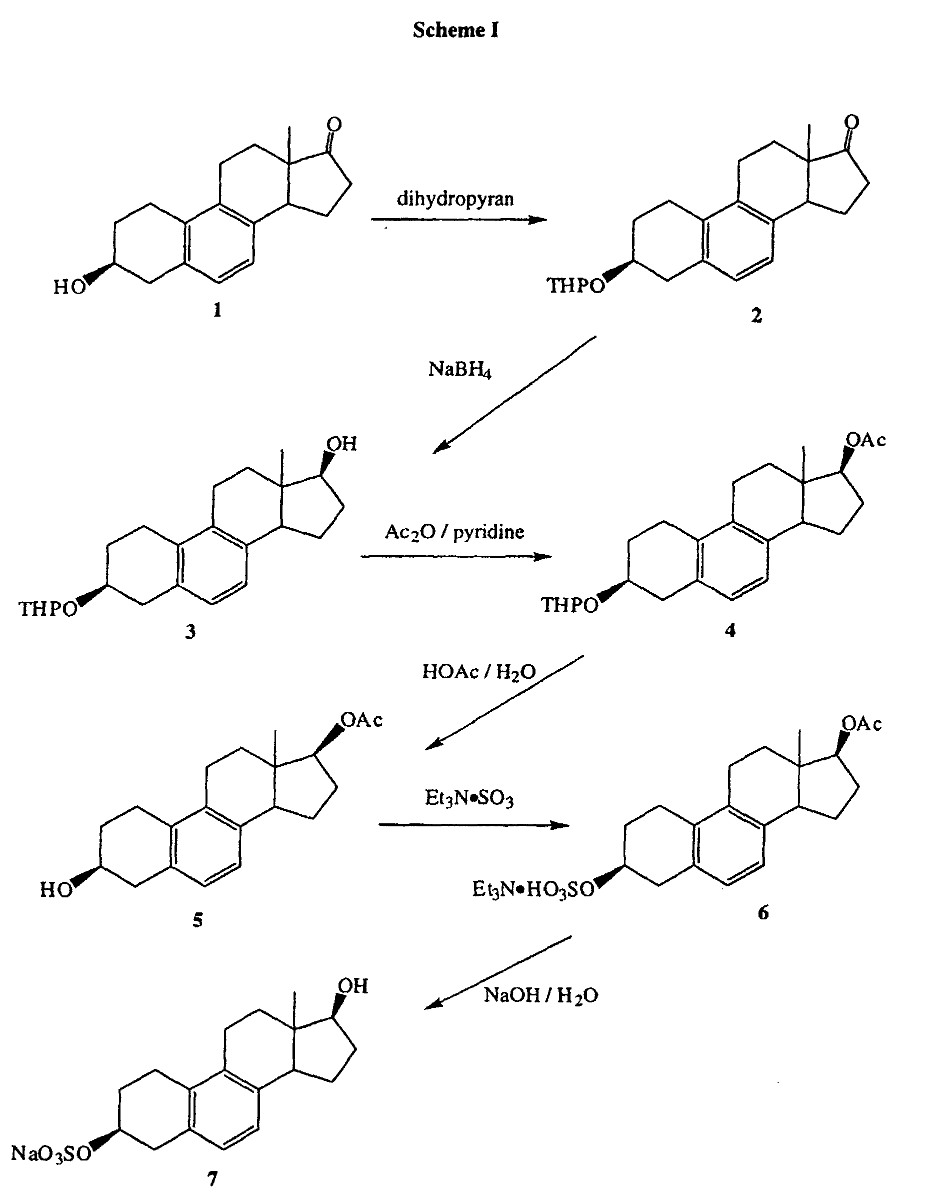

5,7,9-Estratriene-3β,17β-diol 3-sulfate ester sodium salt (**7**) can also be prepared by the selective sulfation of diol (**8**) as shown in Scheme II. Diol (**8**) can be prepared from (**1**) by the reduction of the 17-ketone with a reducing agent such as sodium borohydride.

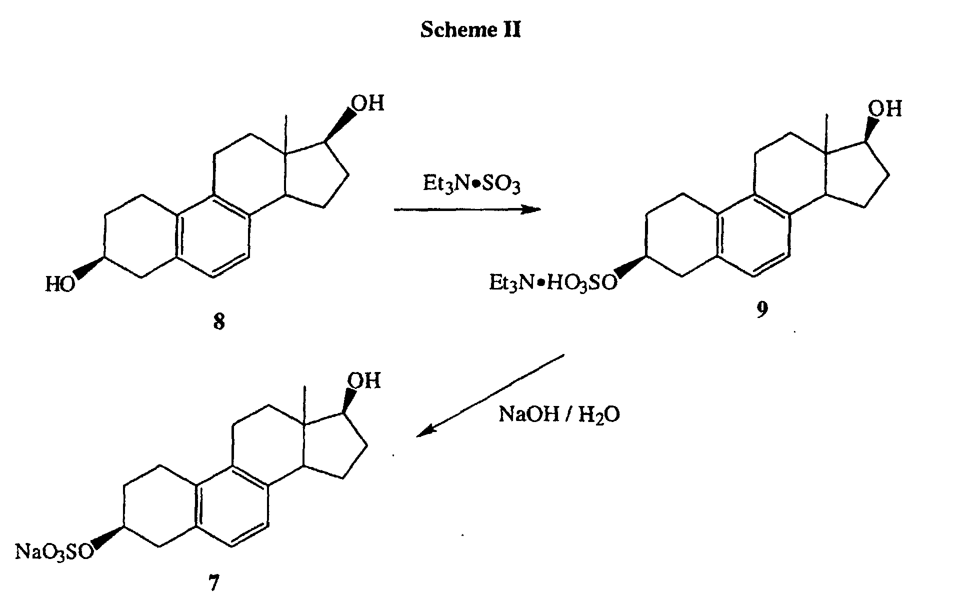

5,7,9-Estratriene-3β,17β-diol 3-sulfate ester sodium salt (**7**) can also be prepared according to Scheme III by the reduction of 3β-hydroxy-5,7,9-estratriene-17-one 3-sulfate ester sodium salt (**10**) with a reducing agent such as sodium borohydride.

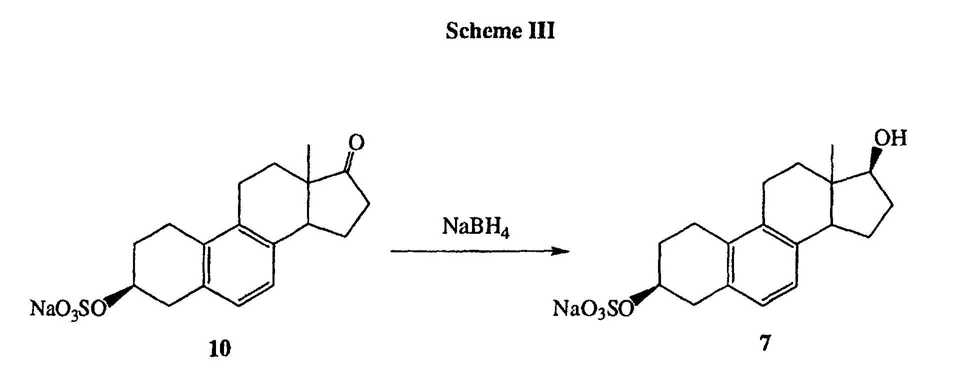

5,7,9-Estratriene-3α,17β-diol 3-sulfate ester sodium salt (**17**) can be prepared according to Scheme IV according to standard literature procedures. The introduction of the 3α-hdyroxyl group can be accomplished by the reduction of the 3-ketone (**14**) with a bulky reducing agent such as lithium tri-t-butoxy aluminumhydride.

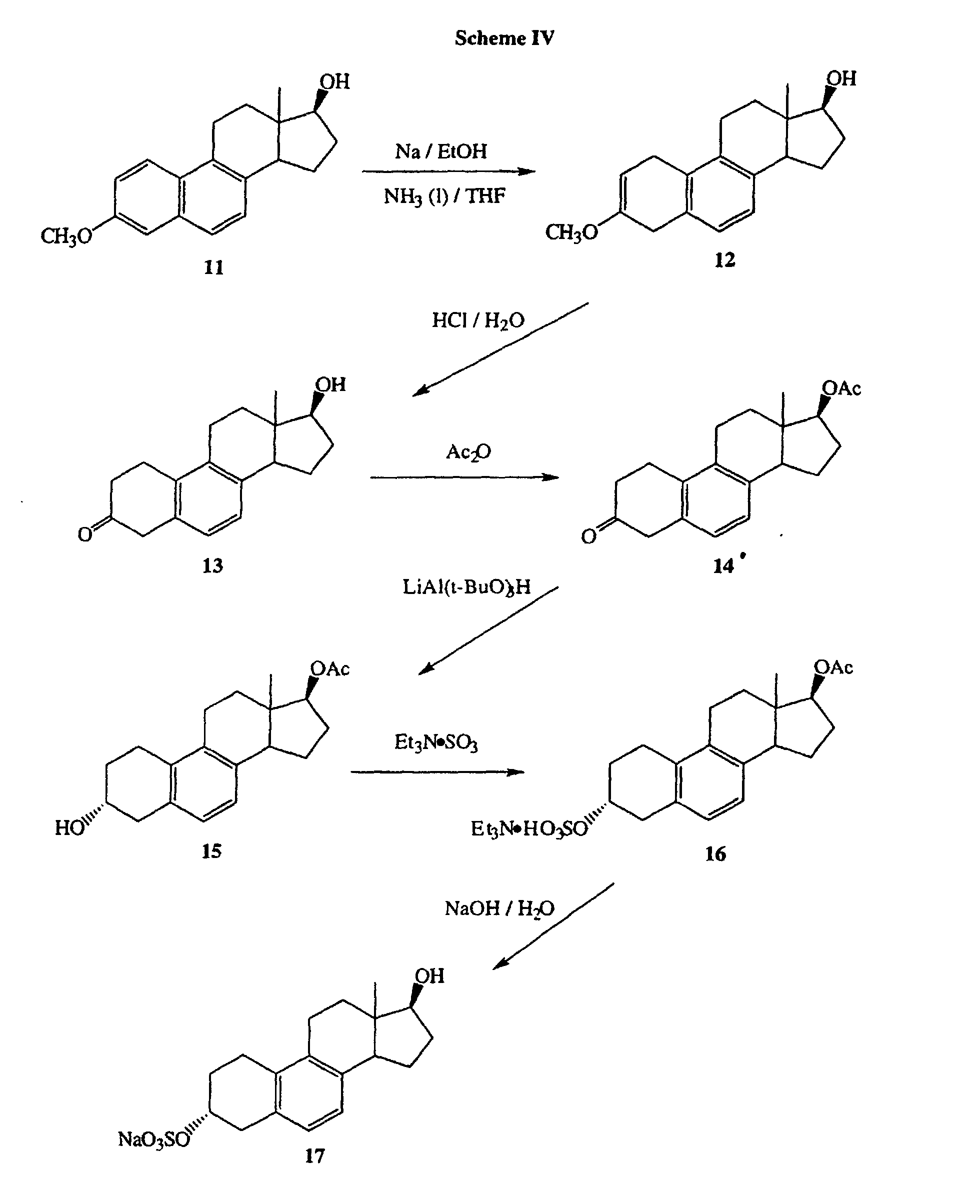

5,7,9-Estratriene-3α,17β-diol 3-sulfate ester sodium salt (**17**) can also be prepared according to Scheme V according to standard literature procedures.

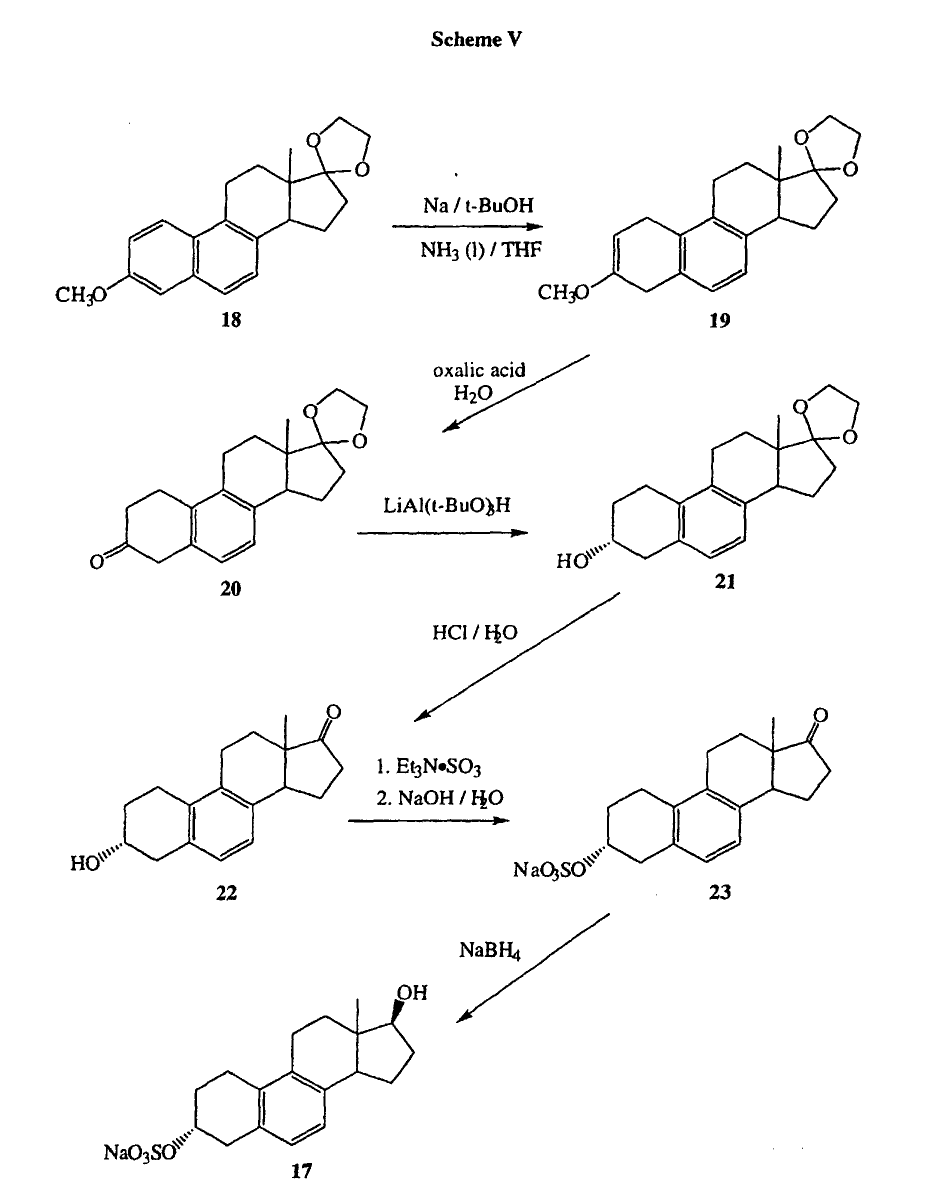

The preparation of 5,7,9-Estratriene-3β,17α-diol 3-sulfate ester sodium salt (**32**) can be prepared by standard methodology as shown in Scheme VI.

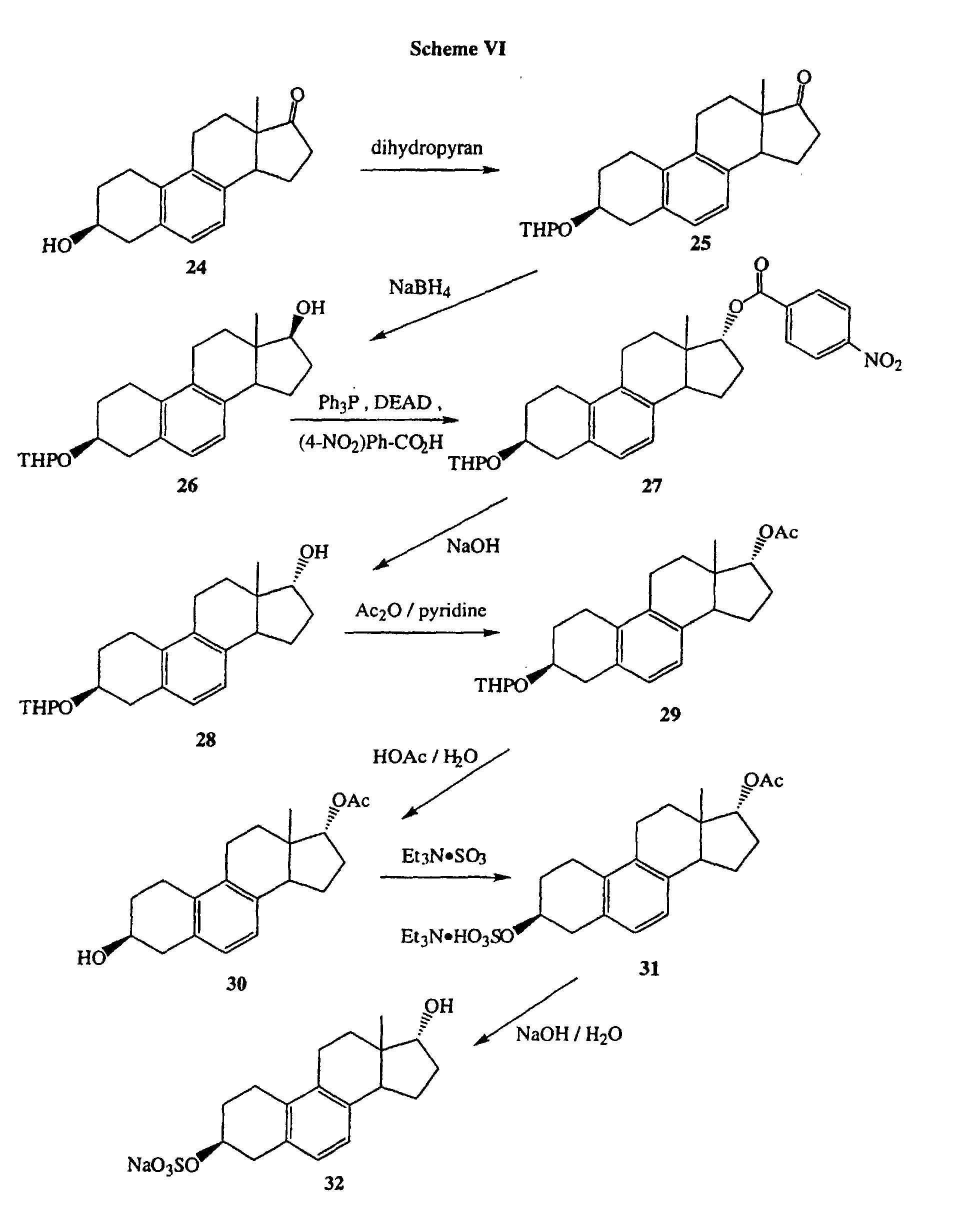

Compounds of this invention having the 3α,17α-configuration can be prepared by stereochemical inversion of the 3β-alcohol. For example, the 3β-hydroxyl group of compound (**30**) can be treated under the conditions used to effect the transformation from (**26**) to (**27**) to provide 3α,17α-stereochemistry. Sulfation hydroxyl groups at the 17-position for compounds of this invention can be accomplished via a protected 3-hydroxyl group or 3-ketone.

The compounds of this invention are estrogenic, as shown in the in vitro and in vivo standard pharmacological test procedures described below in which 5,7,9-estratriene-3β,17β-diol was evaluated as a representative compound of this invention.

### Estrogen Receptor Binding

An initial evaluation examined the competitive binding properties of 5,7,9-estratriene-3β,17β-diol to the human estrogen receptor (hER-α) prepared as a soluble cell extract (cytosol). In this standard pharmacological test procedure, 5,7,9-estratriene-3β,17β-diol demonstrated no specific binding activity. However, when estrogen receptor binding was analyzed using a whole cell test procedure, specific binding was clearly demonstrated. This test procedure indicated an IC₅₀ of 2.3 x 10⁻⁸ M for 5,7,9-estratriene-3β,17β-diol. This would be compared with a Kᵢ for estrone, equilin and equilinen of 51, 67 and 375nM, respectively.

### In Vitro Cotransfection Test Procedure

In this standard pharmacological test procedure, hER-α over-expressed in Chinese hamster ovary (CHO) cells infected with adeno-2x-ERE-tk-luciferase, an estrogen responsive reporter gene construct, cells were exposed to varying concentrations (10⁻¹²-10⁻⁵M) of 5,7,9-estratriene-3β,17β-diol for 24 hours. Cells were also exposed to 17β-estradiol at 10⁻⁹ M. Following the 24-hour treatment, cells were lysed and cell extracts assayed for luciferase activity. The results provided that 5,7,9-estratriene-3β,17β-diol had an EC₅₀ of approximately 30 nM. Using a similar test procedure, previous data indicate a 5.6nM EC₅₀ for estrone.

### In Vivo Uterotropic Activity

Immature rats were treated with doses of 0.2 and 2 mg/kg 5,7,9-estratriene-3β,17β-diol for three days (S.C.) as well as additional groups (n=6) of rats treated with 0.5 µg ethinyl estradiol and vehicle as positive and negative controls, respectively. Rats treated with 5,7,9-estratriene-3β,17β-diol had uterine weight gains at both concentrations demonstrating that 5,7,9-estratriene-3β,17β-diol was estrogenic..

The results of these standard pharmacological test procedures demonstrate that the compounds of this invention are estrogenic.

Based on the results obtained in the standard pharmacological test procedures, the compounds of this invention are useful in providing estrogen replacement therapy following ovariectomy or menopause, and in relieving symptoms related to estrogen deficiency, including vasomotor symptoms, such as hot flushes, and other menopausal related conditions, such as vaginal atrophy, vaginitis, and atrophic changes of the lower urinary tract which may cause increased urinary frequency, incontinence, and dysuria. The compounds of this invention are useful in preventing bone loss and in the inhibition or treatment of osteoporosis. The compounds of this invention are cardioprotective and they are useful in the treatment of atherosclerosis, ischemic disease, and hypertension. These cardiovascular protective properties are of great importance when treating postmenopausal patients with estrogens to prevent osteoporosis and in the male when estrogen therapy is indicated. Additionally, the compounds of this invention are useful in the suppression of lactation, and in the prophylaxis and treatment of mumps orchitis.

The compounds of this invention can be used alone as a sole therapeutic agent or can be used in combination with other agents, such as other estrogens, progestins, or and androgens.

The compounds of this invention can be formulated neat or with a pharmaceutical carrier for administration, the proportion of which is determined by the solubility and chemical nature of the compound, chosen route of administration and standard pharmacological practice. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compounds of this invention can also be administered orally either in liquid or solid composition form.

The compounds of this invention may be administered rectally or vaginally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

The dosage requirements vary with the particular compositions employed, the route of administration, the severity of the symptoms presented and the particular subject being treated. Based on the results obtained in the standard pharmacological test procedures, projected daily dosages of active compound would be 0.02 µg/kg - 750 µg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

## Claims

1. A compound having the formula 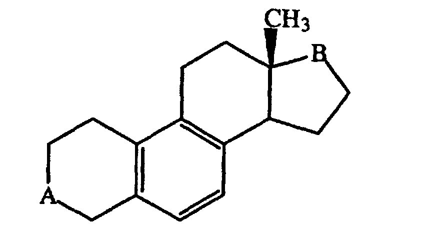 wherein
A and B are each, independently, 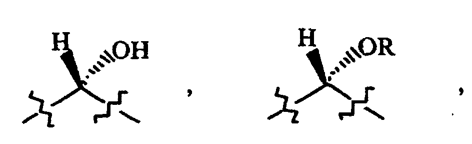 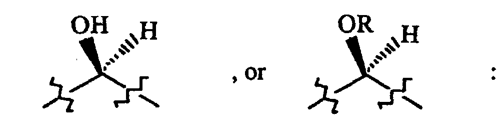
R is SO₃⁻ X⁺ ;
X⁺ is alkali metal, alkaline earth metal, ammonium, alkylammonium containing 1-6 carbon atoms, dialkylammonium containing 1-6 carbon atoms in each alkyl group, trialkylammonium containing 1-6 carbon atoms in each alkyl group or tetraalkylammonium containing 1-6 carbon atoms in each alkyl group;
with the proviso that at least one of A or B is

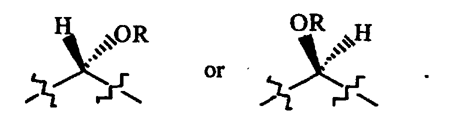

2. 5,7,9-Estratriene-3β,17β-diol 3-sulfate ester sodium salt.

3. A compound as claimed in Claim 1 or Claim 2 for use as a medicament.

4. Use of a compound of Claim 1 or Claim 2 in the preparation of a medicament for providing estrogen replacement therapy or treating estrogen deficiency in a mammal.

5. Use of a compound of Claim 1 or Claim 2 in the preparation of a medicament for treating vasomotor symptoms related to estrogen deficiency in a mammal.

6. Use according to claim 5 wherein the vasomotor symptom is hot flushes.

7. Use of a compound of Claim 1 or Claim 2 in the preparation of a medicament for treating or inhibiting osteoporosis in a mammal.

8. Use of a compound of Claim 1 or Claim 2 in the preparation of a medicament for treating or inhibiting atherosclerosis in a mammal.

9. A pharmaceutical composition which comprises a compound having the formula 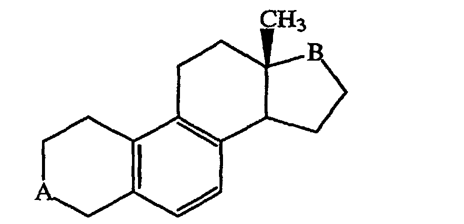 wherein
A and B are each, independently, 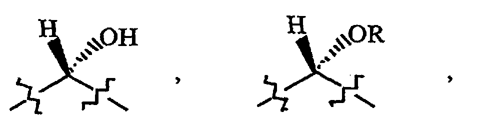 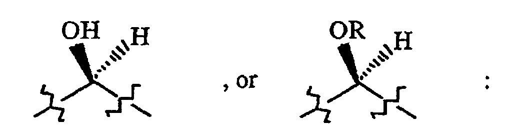
R is SO₃⁻X⁺;
X⁺ is alkali metal, alkaline earth metal, ammonium, alkylammonium containing 1-6 carbon atoms, dialkylammonium containing 1-6 carbon atoms in each alkyl group, trialkylammonium containing 1-6 carbon atoms in each alkyl group or tetraalkylammonium containing 1-6 carbon atoms in each alkyl group;
with the proviso that at least one of A or B is 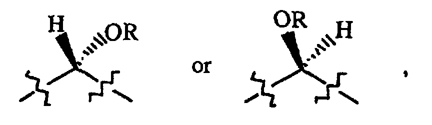 and a pharmaceutical carrier.

10. A pharmaceutical composition according to Claim 9 which comprises at least 1% of a compound as defined in Claim 1 or Claim 2.

11. A process for the preparation of 5,7,9-estratriene-3,17-diol-3,17-disulfate ester salts according to Claim 1 which comprises one of the following:
a) converting the appropriate 5,7,9-estratriene-3,17-diol-3,17-disulfate ester to a pharmaceutically acceptable salt,
b) converting a pharmaceutically acceptable salt of the 5,7,9-estratriene-3,17-diol-3,17-disulfate ester to a different pharmaceutically acceptable salt of the 5,7,9-estratriene-3,17-diol-3,17-disulfate ester, or
c) directly converting the appropriate 5,7,9-estratriene-3,17-diol to the desired pharmaceutically acceptable salt of 5,7,9-estratriene-3,17-disulfate ester.

12. A process for the preparation of 5,7,9-estratriene-3,17-diol-3-sulfate ester salts or 5,7,9-estratriene-3,17-diol-17-sulfate ester salts according to Claim 1 which comprises one of the following:
a) converting the appropriate 5,7,9-estratriene-3,17-diol-mono sulfate ester to a pharmaceutically acceptable salt,
b) converting a pharmaceutically acceptable salt of the appropriate 5,7,9-estratriene-3,17-diol-mono sulfate ester to a different pharmaceutically acceptable salt of the appropriate the appropriate 5,7,9-estratriene-3,17-diol-mono sulfate ester,
c) converting the corresponding 5,7,9-estratiiene-3-ol,17-keto 3 sulfate ester salt or 5,7,9-estratriene-17-ol,3-keto 17 sulfate ester salt to the desired 5,7,9-estratriene-3,17-diol-3-sulfate ester salt or 5,7,9-estratriene-3,17-diol-17-sulfate ester salt,
d) converting 5,7,9-estratriene-3,17-diol to the desired pharmaceutically acceptable salt of the desired 5,7,9-estratriene-3,17-diol-monosulfate ester directly,
e) deprotecting the corresponding 17 protected 5,7,9-estratriene-3,17-diol-3-sulfate ester salt or 3 protected 5,7,9-estratriene-3,17-diol-17-sulfate ester salt, or
f) converting one stereo isomer of the pharmaceutically acceptable salt of the appropriate 5,7,9-estratriene-3,17-diol-mono sulfate ester to another by inversion.

## Patentansprüche

1. Verbindung mit der Formel 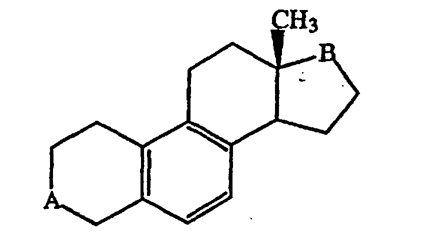 worin
A und B jeweils unabhängig 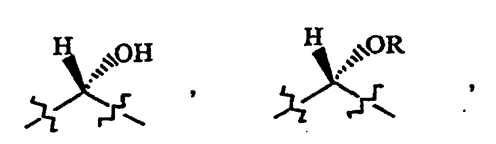 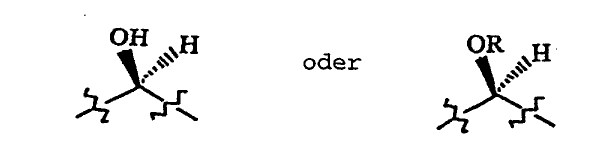 darstellen;
R für SO₃⁻X⁺ steht;
X⁺ Alkalimetall, Erdalkalimetall, Ammonium, Alkylammonium welches 1-6 Kohlenstoffatome enthält, Dialkylammonium welches 1-6 Kohlenstoffatome in jeder Alkylgruppe enthält, Trialkylammonium welches 1-6 Kohlenstoffatome in jeder Alkylgruppe enthält oder Tetraalkylammonium welches 1-6 Kohlenstoffatome in jeder Alkylgruppe enthält, darstellt;
unter der Bedingung, dass mindestens eines von A oder B für 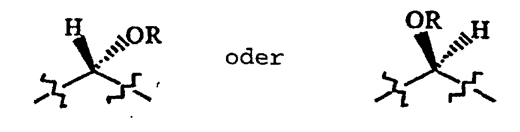 steht.

2. 5,7,9-Estratrien-3β,17β-diol-3-sulfatester-natriumsalz.

3. Verbindung wie in Anspruch 1 oder Anspruch 2 beansprucht, zur Verwendung als Arzneimittel.

4. Verwendung einer Verbindung nach Anspruch 1 oder Anspruch 2 bei der Herstellung eines Arzneimittels zum Vorsehen von Östrogen-Ersatztherapie oder Behandeln von Östrogenmangel in einem Säuger.

5. Verwendung einer Verbindung nach Anspruch 1 oder Anspruch 2 bei der Herstellung eines Arzneimittels um Behandeln vasomotorischer Symptome in Verbindung mit Östrogenmangel in einem Säuger.

6. Verwendung gemäß Anspruch 5, wobei das vasomotorische Symptom Hitzewallungen ist.

7. Verwendung einer Verbindung nach Anspruch 1 oder Anspruch 2 bei der Herstellung eines Arzneimittels zum Behandeln oder Hemmen von Osteoporose in einem Säuger.

8. Verwendung einer Verbindung nach Anspruch 1 oder Anspruch 2 bei der Herstellung eines Arzneimittels zum Behandeln oder Hemmen von Atherosklerose in einem Säuger.

9. Pharmazeutische Zusammensetzung, welche umfasst: eine Verbindung mit der Formel 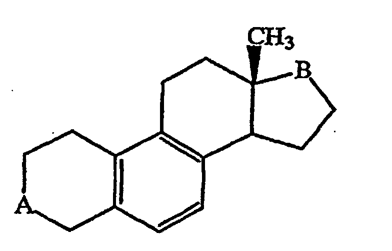 worin
A und B jeweils unabhängig 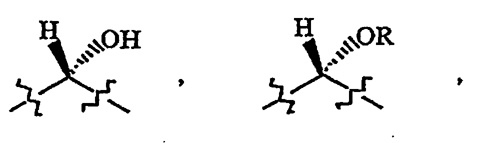 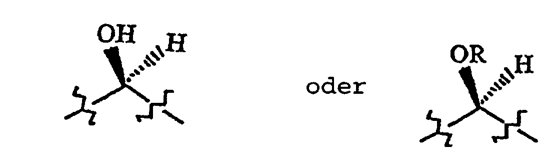 darstellen;
R für CO₃⁻X⁺ steht;
X⁺ Alkalimetall, Erdalkalimetall, Ammonium, Alkylammonium welches 1-6 Kohlenstoffatome enthält, Dialkylammonium welches 1-6 Kohlenstoffatome in jeder Alkylgruppe enthält, Trialkylammonium welches 1-6 Kohlenstoffatome in jeder Alkylgruppe enthält oder Tetraalkylammonium welches 1-6 Kohlenstoffatome in jeder Alkylgruppe enthält, darstellt;
unter der Bedingung, dass mindestens eines von A oder B für 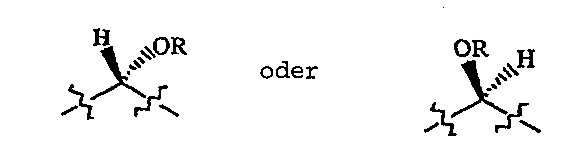 steht, und einen pharmazeutischen Träger.

10. Pharmazeutische Zusammensetzung gemäß Anspruch 9, welche mindestens 1% einer Verbindung umfasst, wie in Anspruch 1 oder Anspruch 2 definiert.

11. Verfahren für die Herstellung von 5,7,9-Estratrien-3,17-diol-3,17-disulfatestersalzen gemäß Anspruch 1, welches eines der folgenden umfasst:
a) Umwandeln des passenden 5,7,9-Estratrien-3,17-diol-3,17-disulfatesters in ein pharmazeutisch annehmbares Salz,
b) Umwandeln eines pharmazeutisch annehmbaren Salzes des 5,7,9-Estratrien-3,17-diol-3,17-disulfatesters in ein anderes pharmazeutisch annehmbares Salz des 5,7,9-Estratrien-3,17-diol-3,17-disulfatesters, oder
c) direktes Umwandeln des passenden 5,7,9-Estratrien-3,17-diols in das gewünschte pharmazeutisch annehmbare Salz von 5,7,9-Estratrien-3,17-disulfatester.

12. Verfahren für die Herstellung von 5,7,9-Estratrien-3,17-diol-3-sulfatestersalzen oder 5,7,9-Estratrien-3,17-diol-17-sulfatestersalzen gemäß Anspruch 1, welches eines der folgenden umfasst:
a) Umwandeln des passenden 5,7,9-Estratrien-3,17-diolmonosulfatesters in ein pharmazeutisch annehmbares Salz,
b) Umwandeln eines pharmazeutisch annehmbaren Salzes des passenden 5,7,9-Estratrien-3,17-diol-monosulfatesters in ein anderes pharmazeutisch annehmbares Salz des passenden 5,7,9-Estratrien-3,17-diol-monosulfatesters,
c) Umwandeln des entsprechenden 5,7,9-Estratrien-3-ol,17-keto-3-sulfatestersalzes oder 5,7,9-Estratrien-17-ol,3-keto-17-sulfatestersalzes in das gewünschte 5,7,9-Estratrien-3,17-diol-3-sulfatestersalz oder 5,7,9-Estratrien-3,17-diol-17-sulfatestersalz,
d) Umwandeln von 5,7,9-Estratrien-3,17-diol in das gewünschte pharmazeutisch annehmbare Salz des gewünschten 5,7,9-Estratrien-3,17-diol-monosulfatesters direkt,
e) Entschützen des entsprechenden 17-geschützten 5,7,9-Estratrien-3,17-diol-3-sulfatestersalzes oder 3-geschützten 5,7,9-Estratrien-3,17-diol-17-sulfatestersalzes, oder
f) Umwandeln eines Stereoisomers des pharmazeutisch annehmbaren Salzes des passenden 5,7,9-Estratrien-3,17-diolmonosulfatesters in ein anderes durch Inversion.

## Revendications

1. Composé de formule : 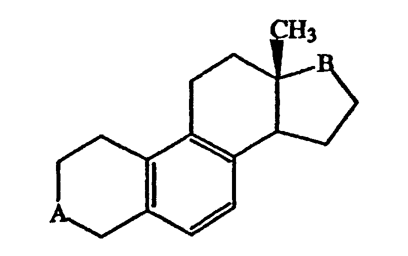 dans laquelle
A et B sont chacun indépendamment : 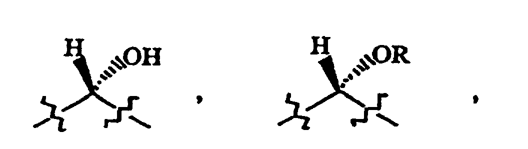 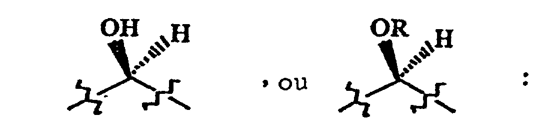
R est SO₃⁻X⁺;
X⁺ est un métal alcalin, un métal alcalino-terreux, de l'ammonium, un alkylammonium contenant 1 à 6 atomes de carbone, un dialkylammonium contenant 1 à 6 atomes de carbone dans chaque groupement alkyle, un trialkylammonium contenant 1 à 6 atomes de carbone dans chaque groupement alkyle ou un tétraalkylammonium contenant 1 à 6 atomes de carbone dans chaque groupement alkyle;
pourvu qu'au moins l'un des groupements A et B soit :

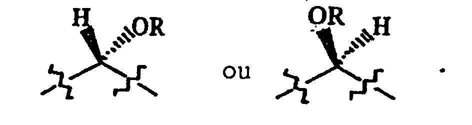

2. Sel sodique de l'ester 3-sulfate de 5,7,9-oestratriène-3β,17β-diol.

3. Composé selon la revendication 1 ou 2 pour un usage comme médicament.

4. Utilisation d'un composé selon la revendication 1 ou 2 pour la préparation d'un médicament afin d'effectuer une oestrogénothérapie de substitution ou le traitement d'un déficit en oestrogènes chez un mammifère.

5. Utilisation d'un composé selon la revendication 1 ou 2 pour la préparation d'un médicament pour le traitement de symptômes vasomoteurs liés à un déficit en oestrogènes chez un mammifère.

6. Utilisation selon la revendication 5, dans laquelle le symptôme vasomoteur consiste en bouffées de chaleur.

7. Utilisation d'un composé selon la revendication 1 ou 2 pour la préparation d'un médicament pour le traitement ou l'inhibition de l'ostéoporose chez un mammifère.

8. Utilisation d'un composé selon la revendication 1 ou 2 pour la préparation d'un médicament pour le traitement ou l'inhibition de l'athérosclérose chez un mammifère.

9. Composition pharmaceutique qui comprend un composé ayant pour formule : 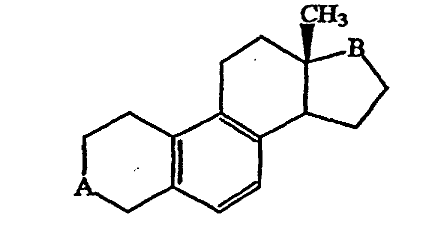 dans laquelle
A et B sont chacun indépendamment : 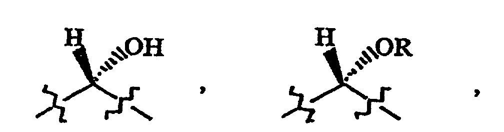 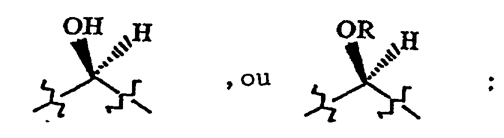
R est SO₃⁻X⁺;
X⁺ est un métal alcalin, un métal alcalino-terreux, de l'ammonium, un alkylammonium contenant 1 à 6 atomes de carbone, un dialkylammonium contenant 1 à 6 atomes de carbone dans chaque groupement alkyle, un trialkylammonium contenant 1 à 6 atomes de carbone dans chaque groupement alkyle ou un tétraalkylammonium contenant 1 à 6 atomes de carbone dans chaque groupement alkyle;
pourvu qu'au moins l'un des groupements A et B soit : 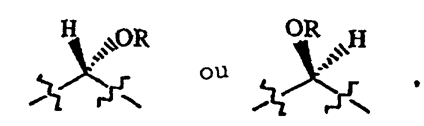 et un véhicule pharmaceutique.

10. Composition pharmaceutique selon la revendication 9, qui comprend au moins 1% d'un composé défini selon la revendication 1 ou 2.

11. Procédé pour la préparation de sels d'esters 3,17-disulfate de 5,7,9-oestratriène-3,17-diol selon la revendication 1, qui comprend l'un des suivants:
a) la conversion de l'ester 3,17-disulfate de 5,7,9-oestratriène-3,17-diol en un sel pharmaceutiquement acceptable,
b) la conversion d'un sel pharmaceutiquement acceptable de l'ester 3,17-disulfate de 5,7,9-oestratriène-3,17-diol en un sel pharmaceutiquement acceptable différent de l'ester 3,17-disulfate de 5,7,9-oestratriène-3,17-diol,
c) la conversion directe du 5,7,9-oestratriène-3,17-diol en le sel pharmaceutiquement acceptable de l'ester 3,17-disulfate de 5,7,9-oestratriène.

12. Procédé pour la préparation de sels d'esters 3-sulfate de 5,7,9-oestratriène-3,17-diol ou de sels d'esters 17-sulfate de 5,7,9-oestratriène-3,17-diol selon la revendication 1, qui comprend l'un des suivants :
a) la conversion de l'ester monosulfate de 5,7,9-oestratriène-3,17-diol approprié en un sel pharmaceutiquement acceptable,
b) la conversion d'un sel pharmaceutiquement acceptable de l'ester monosulfate de 5,7,9-oestratriène-3,17-diol approprié en un sel pharmaceutiquement acceptable différent de l'ester monosulfate de 5,7,9-oestratriène-3,17-diol,
c) la conversion du sel d'ester 17-céto-3-sulfate de 5,7,9-oestratriène-3-ol ou du sel d'ester 3-céto-17-sulfate de 5,7,9-oestratriène-17-ol en le sel d'ester 3-sulfate de 5,7,9-oestratriène-3,17-diol souhaité ou en le sel d'ester 17-sulfate de 5,7,9-oestratriène-3,17-diol souhaité,
d) la conversion directe de 5,7,9-oestratriène-3,17-diol en le sel pharmaceutiquement acceptable souhaité de l'ester monosulfate de 5,7,9-oestratriène-3,17-diol souhaité,
e) la déprotection du sel d'ester 3-sulfate de 5,7,9-oestratriène-3,17-diol correspondant protégé en 17 ou du sel d'ester 17-sulfate de 5,7,9-oestratriène-3,17-diol correspondant protégé en 3, ou
f) la conversion d'un stéréoisomère du sel pharmaceutiquement acceptable de l'ester monosulfate de 5,7,9-oestratriène-3,17-diol approprié en un autre par inversion.
